# EUROPEAN PATENT SPECIFICATION

(11) **EP 2 000 484 B1**
(45) Date of publication and mention of the grant of the patent: **30.11.2011**
(21) Application number: 06730145.7
(22) Date of filing: 27.03.2006
(51) Int. Cl.: C08F 2/24, C08F 2/44, C08F 12/06, C08F 12/36, C08F 20/32, C08K 3/22, C08L 57/00

(54) **PROCESS FOR PRODUCING POLYMER-COATED FERROMAGNETIC PARTICLE, AND POLYMER-COATED FERROMAGNETIC PARTICLE**
VERFAHREN ZUR HERSTELLUNG VON POLYMERBESCHICHTETEM FERROMAGNETISCHEM TEILCHEN UND POLYMERBESCHICHTETES FERROMAGNETISCHES TEILCHEN
PROCEDE DE PRODUCTION DE PARTICULE FERROMAGNETIQUE ENDUITE DE POLYMERE ET PARTICULE FERROMAGNETIQUE ENDUITE DE POLYMERE

(43) Date of publication of application: 10.12.2008
(73) Proprietor: Tokyo Institute of Technology, Tokyo 152-8550 (JP); Tamagawa Seiki Kabushiki Kaisha, Iida-shi, Nagano 395-8515 (JP)
(72) Inventor: HANDA, Hiroshi, Yokohama-shi, Kanagawa 2268503 (JP); NISHIO, Kosuke, Yokohama-shi, Kanagawa 2268503 (JP); GOKON, Nobuyuki, Yokohama-shi, Kanagawa 2268503 (JP); ABE, Masanori, Meguro-ku, Tokyo 1528550 (JP)
(74) Representative: Kramer - Barske - Schmidtchen
(86) International application number: PCT/JP2006/306197
(87) International publication number: WO 2007/110917

(56) References cited:
- EP-A1- 0 038 730
- EP-A1- 0 054 832
- EP-A1- 0 390 634
- JP-A- 02 286 729
- JP-A- 06 102 708
- JP-A- 11 191 510
- JP-A- 2005 200 643
- JP-A- 2005 200 643
- JP-A- 2006 088 131

## Description

### Technical field

The present invention relates to a method of manufacturing ferromagnetic particles having excellent polymer coating showing good response sensitively to magnetic field, to the polymer coated ferromagnetic particles, and to usage of the polymer coated ferromagnetic particles.

### Background Art

Materials showing ferromagnetic characteristics in bulk state such as ferrite materials do not show ferromagnetic characteristics but show super-paramagnetic behavior when the particle size is very small. Furthermore, when the particle size is equal to or larger than a critical size, the materials show ferromagnetic behavior and apt to form particle aggregates due to the magnetic attractive force between particles. When aggregates are formed, the particles behave like particles with effectively larger particle size, and are prone to yield particle dispersion instability in liquid. Then various inconveniences for applying the particles occur. There are many efforts of using the particles to applications avoiding aggregate formation by weakening the cohesive force between the ferromagnetic particles applying various methods. The methods of coating the ferromagnetic particles with polymer to provide steric hindrance to cope with the attractive forces between the ferromagnetic particles, and methods of providing electrostatic repulsive force between functional groups are examples of the methods. The polymer coated ferromagnetic particles are widely used in many industrial fields, for example as magnetic carrier of the toner for electro-photography, magnetic colloid, magnetic ink, and magnetic paint can be cited. Furthermore, the particles are widely used as raw materials of molded products, for example, as raw particles for molded plastic magnets.

However, problems are pointed out to the existing polymer coated ferromagnetic particles of relatively large particle sizes of micron meter order with large particle size scattering. There were little or no methods for controlling polymer coating thickness and shape, especially for particles having average diameter of 500 nm or less. Therefore, a new technology that can attain the control has been desired. In biological science and medical activity in recent years, polymer coated magnetic particles such as polymer coated ferrite particles, are used as a carrier of a bio-separation, and a magnetic marker for bio-molecules. In these applications also, a new method of obtaining thickness and shape controlled coated particles having average diameter of 500 nm or less with good particle diameter uniformity has been desired.

Inventors of the present invention developed bonded composite fine particles of polymer particles and ferrite fine particles (Patent reference 1: JP-A 2002-090366(KOKAI)), and also developed ferrite particles bonded with organic material in which the organic material are directly bonded to the ferrite particles (Patent reference 2: WO 03/066644 A1), and have shown there that these have desirable characteristics in usage of purification and identification for the specific drug substances.

A research work related to the work is found for example in "Scientific and Clinical Applications of Magnetic Carriers, Plenum Press 1997" (Non-patent reference 1), in which polymer fine particles bonded to ferrite having magnetic response and their application are described.

A patent publication for coating inorganic fine particles such as ferrite particles with polymer and introducing functional groups that react with biological molecules or chemical molecules on the polymer coats and applying the particles for detecting, separating and refining specified bio-molecules using the specific interaction characteristics of the molecules is found in JP-A 2003-513093(KOKAI) (Patent reference 3).

When ferrite particles are applied as ferromagnetic particles of polymer coated ferromagnetic particles, the particle diameter is desired to be at an extent of 100 nm or less than 100 nm for sustaining colloidal dispersed state in liquid. The particles, on the other hand, are hoped to have magnetization sufficient to respond to handling using magnetic field. For this purpose, the particle size is required not to be too small such that the particles show very small magnetization per volume, for example the particle characteristics designed to have average diameter of equal to or larger than 30 nm. The magnetization decrease of ferrite particles due to theirminiaturization involves nonmagnetic surface layer. The fraction of the nonmagnetic surface layer increases when the diameter becomes small and result in rapid decrease of magnetization. As the particle size become small, the particles show single magnetic domain structure in the first place, and then fluctuation of the magnetization direction due to thermal fluctuation effect occurs.

In each application of the polymer coated ferromagnetic particles, the surfaces of the ferromagnetic particles are desired to be covered sufficiently with polymer. In addition, the polymer coating is desired to form bonding between polymer and ferromagnetic particles such as ferrite particles tight enough to avoid dissociation during use of the particles.

Core ferromagnetic particles are desired to have large magnetization to obtain polymer coated ferromagnetic particles with good response to magnetic field for handling. However, the particles tend to flocculate strongly as a result of strong magnetic interaction between particles when the ferromagnetic particles have large magnetization. Therefore, it was difficult to obtain sufficiently dispersed fine polymer coated ferromagnetic particles in which each particle is coated with polymer.

When the ferromagnetic particles are ferrite particles, the particle size is desirable to be 20 nm or more, and is more desirable to be 30 nm or more for obtaining sufficient magnetization. However, it is very difficult to disperse ferrite particles having particle diameter of 30 nm or more, because the attractive force between the particles is strong enough. Then it has been difficult to obtain sufficiently dispersed fine ferrite particules in which each particle was coated with polymer. For this reason, known polymer coated ferrite particles having an extent of diameters of about 10 nm or less are limited to particles the type in which each aggregate of particles is coated as a whole, and the type in which coated particles of very small diameter ferrite particles with very weak magnetic aggregating force having very weak response to magnetic field because the particles have very small magnetization.
[Patent reference 1] JP-A 2002-090366(KOKAI)
[Patent reference 2] WO 03/066644 A1
[patent reference 3] JP-A 2003-513093(KOKAI)
[Non-patent reference 1] Scientific and Clinical Applications of Magnetic Carriers, Plenum Press 1997
JP 2005-200643 A relates to a method for manufacturing polymer-coated minute particles and to polymer-coated minute particles.
EP 0 390 634 A1 relates to magnetizable composite microspheres of a hydrophobic crosslinked vinylaromatic polymer having a diameter of about 0.05 to 10 µm, comprising (1) a core comprising magnetizable particles less than 300 x 10⁻⁴ µm in diameter coated with an approximately monomolecular layer of a non-water soluble dispersing agent, and (2) a shell consisting essentially of a hydrophobic crosslinked copolymer formed from at least one hydrophobic vinylaromatic monomer and at least one polyethylenically unsaturated emulsifying polymer which is soluble in said vinylaromatic monomer(s) and capable of crosslinking with said vinylaromatic monomer(s), wherein said shell is substantially free from said magnetic particles relative to said core.
EP 0 038 730 A1 relates to a magnetic-polymer latex, comprising (a) less than 65 % by weight of hydrophobic vinyl aromatic polymer particles having a mean diameter between 0.03 and 5 microns, and (b) a magnetically-charged material in an amount from 0.5 to 50 % by weight with respect to the polymer portion of said particles, said magnetically-charged material being dispersed within said polymer particles.
EP 0 054 832 A1 relates to a process for preparing a colloidal size particulate, comprising the steps of (1) emulsifying at least one hydrophobic, emulsion polymerizable monomer in an aqueous colloidal dispersion of discrete particles of an inorganic solid, said dispersion being maintained in a reasonably stable state with a chemical dispersant and/or surfactant and (2) subjecting the resulting emulsion to emulsion polymerisation conditions to form a stable, fluid aqueous colloidal dispersion of the particulate, wherein essentially every particle of the inorganic solid is coated with the hydrophobic polymer resulting from said polymerization such that substantially all of the inorganic particles are maintained in a discrete spaced apart relationship to each other by the hydrophobic polymer.

### Disclosure of the Invention

This invention presents manufacturing method for obtaining polymer coated ferromagnetic particles having small particle size and showing good response to magnetic field, solving the existing technical problem of dispersing and polymer coating ferromagnetic particles having strong attractive force, and presents highly dispersed polymer coated ferromagnetic particles showing good response to magnetic field.

In particular, this invention provides a method of manufacturing polymer coated ferromagnetic particles as claimed in claim 1 as well as polymer coated ferromagnetic particles obtainable by said method as claimed in claim 8. Further developments of the present invention are set out in the dependent claims.

As a result of concentrated research works of the the present invention, they attained at the present invention finding that sufficiently dispersed polymer coated ferromagnetic particles could be obtained by providing hydrophobic character to ferromagnetic particles and the ferromagnetic particles having hydrophobic character were mixed in water and emulsifying to obtain emulsion liquid with surface activating agents as mentioned above and by emulsion polymerizing the emulsion liquid, even if the ferromagnetic particles had average diameter of 20 nm or more and the particles have magnetic adhesive force showing ferromagnetic characteristics. When the hydrophilic ferromagnetic particles were ferrite particles, it was found that sufficiently dispersed polymer coated ferrite particles can be obtained using this method, even if the ferromagnetic particles have average particle diameter of 30 nm or more. When average diameter of the ferromagnetic particles is larger than 300 nm, the character of the ferromagnetic particles at the emulsifying process changes significantly. Therefore, it is found that the method of this invention is suited to coating ferromagnetic particles having average particle diameter of 300 nm or less. The monomer described above can be monomer in liquid state in itself or monomer liquid as a solution of organic solvent. The monomer can also be a monomer mix containing initiator.

In the present invention, especially good results are obtained, since a nonionic surface activating agent and a ionic surface activating agent are used in combination.

The polymer coated ferromagnetic particles of the present invention can be particles having average diameter of from 25 to 400 nm, comprising hydrophilic ferrite particles having average particle diameter of 20 to 300 nm, an aliphatic acid as a hydrophobizing agent having hydrophilic group and hydrophobic group to provide hydrophobic characteristics to the ferrite particles adsorbing to the ferrite particles through the hydrophilic group of the agent, and polymer coat to adsorb to the hydrophobizing agent and coat the hydrophobized ferrite particles.

Polymer coated ferromagnetic particles that are used in organic solvent according to the present invention are polymer coated ferromagnetic particles durable to organic solvent as explained above. The magnetic solid phase carrier particles for combinatorial chemistry, the magnetic solid carrier particles for affinity chromatography of protein, peptide, or nucleic acid, and magnetic solid phase carrier particles for chemical synthesis of peptide or nucleic acid of the present invention are characterized by using these polymer coated ferromagnetic particles.

The magnetic solid carrier particles for combinatorial chemistry, the magnetic carrier particles for affinity chromatography, and the magnetic carrier particles for chemical synthesis having such construction can have significant solvent durability resistive to the dissolution to the solvents and resistive to the polymer desorption from the particles keeping the polymer coating stable even if the particles are immersed into organic solvents including methanol, ethanol, isopropanol, tetrahydrofuran, acetonitrile, ethyl acetate, dioxane, N,N-dimethylformamide, dimethylsulf oxide, acetone, diethylether, toluene, dicloromethane, chloroform and hexane. The particles can have an advantage of using in organic solvent for magnetic separation since the particles having magnetization are solvent resistive. The particles, therefore, can be used as carrier that can work as stable and quick separation process of combinatorial chemistry, affinity chromatography and chemical synthesis.

As a result of the present invention, a problem of difficulty for dispersing relatively large size ferromagnetic particles having strong cohesive force is solved, and homogeneous and stable polymer coating of ferromagnetic particles such as ferrite particles sufficiently dispersed to monodispersed state or almost monodispersed state can be realized. The particles can be used for various applications including bio-separation in fields of bio-science and clinical medicine, since the polymer coated ferromagnetic particles obtained applying the present invention can have very small particle diameter, can have stable polymer coating and can show good magnetic field response. In each industrial field, for example, significant characteristics increase in magnetic colloid, magnetic ink and magnetic paint can be obtained due to the distinctive character showing good response to magnetic field and stability.

### Brief Description of Drawings

[FIG. 1] FIG. 1 shows a process flow of an embodiment for manufacturing polymer coated ferromagnetic particles according to the present invention.
[FIG. 2] FIG.2 shows an embodiment of the emulsifying process shown in FIG.1 as a flow.
[FIG. 3] FIG.3 shows an embodiment of the process for providing hydrophilic character shown in FIG.2 as a flow.
[FIG. 4A] FIG. 4A is another embodiment of the emulsifying process shown in FIG. 1 as a flow.
[FIG. 4B] FIG.4B is a yet other embodiment of the emulsifying process shown in FIG. 1.
[FIG. 5] FIG. 5 schematically shows an embodiment of the process for coating polymer to ferromagnetic particles partially.
[FIG. 6] FIG.6 schematically shows an embodiment of the process for coating polymer to ferromagnetic particles partially.
[FIG. 7] FIG. 7 schematically shows an embodiment of polymer coated ferromagnetic particles.
[FIG. 8] FIG. 8 shows an electron microscope photographs for an embodiment of polymer coated ferromagnetic particles.

### Description of Symbols

100...hydrophobizing process, 110... ferromagnetic particles, 120...hydrophobizing material, 130... hydrophobic ferromagnetic particles, 200... emulsifying process, 202... first hydrophilic ferromagnetic particles, 203... first process for providing hydrophilic characteristics, 204... second hydrophilic ferromagnetic particles, 205... the second process for providing hydrophilic characteristic, 210... process for providing hydrophilic characteristics, 211... nonionic and ionic surface activating agent, 212... hydrophilic ferromagnetic particles, 213... nonionic surface activating agent, 214... ionic surface activating agent, 220...monomer adding and mixing process, 221... monomer liquid, 222... monomer emulsifying process, 223... monomer emulsion liquid, 224... mixing process, 230... hydrophobic initiator, 240... sonication, 250... water, 260... emulsion liquid 300... emulsion polymerization process, 310... water, 320... sonication, 330...heating, 340...hydrophilic initiator, 350...rinsing process, 360...polymer coated ferrite particles, 360a...polymer coat, 710... epoxy group, 720...amino group, 730... ethylene glycol glycidylether (EGDE).

### Best Mode for Carrying out the Invention

Embodiments of the present invention will be explained specifically below with reference to figures. The description given below shows as examples specific embodiments of the present invention and the description does not limit the scope of the present invention.

### (1) Polymer coating process for ferromagnetic Particles

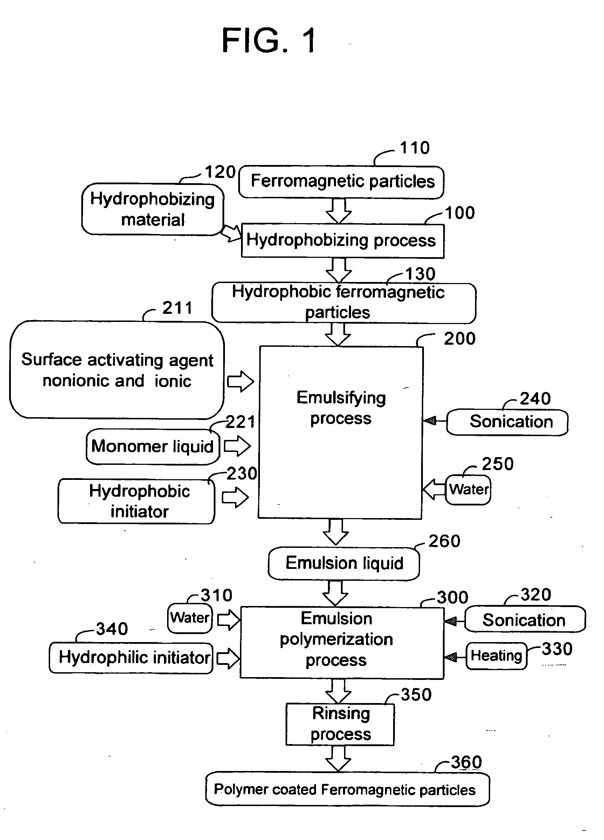

FIG.1 shows a process flow of an embodiment for manufacturing polymer coated ferromagnetic particles. To begin with the hydrophobizing process 100 of FIG.1, the hydrophobic ferromagnetic particles 130 are obtained by adding an aliphatic acid as a hydrophobizing material 120 to adsorb to the ferromagnetic particles 110. At the emulsifying process 200, nonionic surface activating agent having nonionic hydrophilic group such as polyethylene oxide (PEO) chain and ionic surface activating agent having cationic hydrophilic ion group 211 such as primary amine or anionic group such as carboxyl group are mixed with the hydrophobic ferromagnetic particles 130, and monomer liquid 221 containing monomer carrying functional group having potential of radical addition polymerization such as styrene and glycidyl metacrylate (GMA), for example, with organic solvent as needed, is added with water 250 to the hydrophobic ferromagnetic particles. The mixture is then emulsified and emulsion-polymerized. Especially good result is obtained by controlling polymer-coating formation to the ferromagnetic particles of the emulsion polymerization process. In the monomer liquid, hydrophobic initiator 230 such as azobisisobutylonitrile (AIBN) is added as needed. To the monomer liquid 221, cross-linking agent such as divinylbenzene for example is added as needed. Adequate quantity of water 250 is added as needed. Emulsion liquid 260 is obtained mixing and emulsifying the mixture. For the emulsification at the emulsification process 200, sonication 240 of giving ultra sound vibration is proved useful.

At the next process of emulsion polymerization process 300, the emulsion liquid 260 after sonification is heated at temperature equal to or lower than 100°C, desirably at 50 to 90°C, and more desirably at 6 to 80°C, and then hydrophilic initiator is added to polymerize the monomer in the emulsion. In the emulsion polymerization process, it is inferable that the polymerization of monomers proceeds various interactions between monomers inside an emulsion particle, and outside the emulsion particle and monomers between another emulsion particle. The length of the polymerization time period can be set arbitrarily and it may proceed polymerization for about 24 hours.

After removing the surface activating agent by rinsing the polymerized emulsion particles, polymer coated ferromagnetic particles can be obtained.

The emulsifying process 200 explained above and shown in FIG. 1 can be proceeded dividing so as to divide in two processes of process for providing hydrophilic character 210 and monomer adding and mixing process 220 to be processed sequentially.

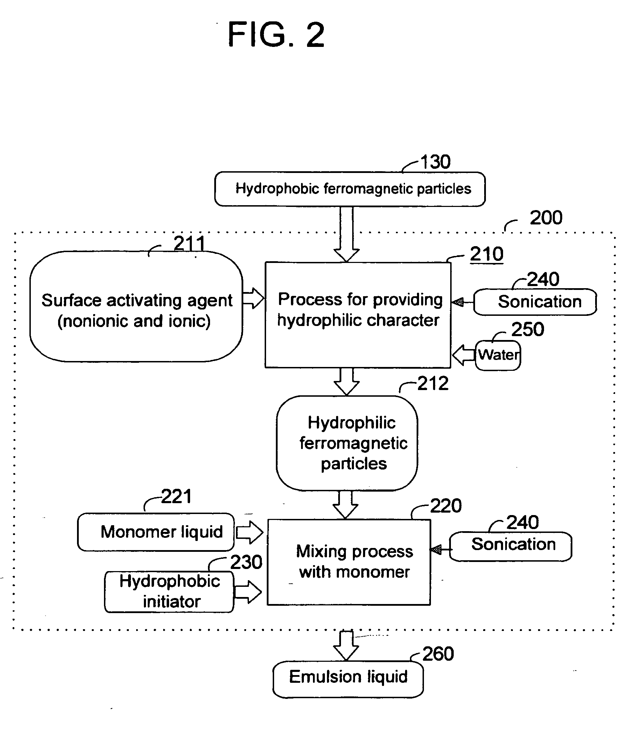

At the process for providing hydrophilic character 210 shown in FIG.2,hydrophilicferromagnetic particles 212 dispersion liquid is obtained by adding and adsorbing surface activating agent 211 containing nonionic surface activating agent and ionic surface activating agent to hydrophobic ferromagnetic particles 130 and further by dispersing, supplying adequate quantity of water 250 as needed. The monomer adding and mixing process 220 can be constructed such that the emulsion liquid 260 is obtained by adding, mixing and emulsifying the monomer liquid 221 explained above to the dispersion liquid of hydrophilic ferromagnetic particles 212. In these processes, sonication 240 providing ultrasonic vibration can be used.

In these way, polymer coated ferromagnetic particles with desirable polymer coating formed at each particle of the hydrophobic ferromagnetic particles 130 can be obtained as a result of polymerizing at the emulsion polymerization process the emulsified emulsion liquid 260.

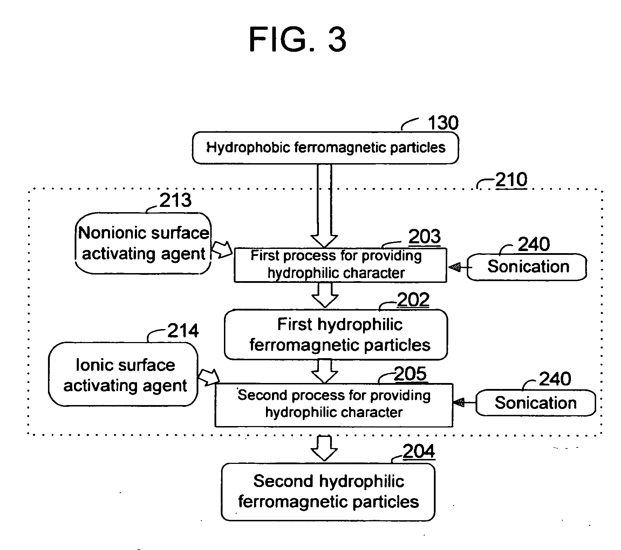

The process for providing hydrophilic character 210 shown in FIG.2 is desirable to be divided to the two processes. The first process for providing hydrophilic character 203 is a process for obtaining the first hydrophilic ferromagnetic particles 202 adsorbing nonionic surface activating agent 213 to the hydrophobic ferromagnetic particles 130. The second process for providing hydrophilic character 205 is a process for obtaining the second hydrophilic ferromagnetic particles 204 adsorbing ionic surface activating agent 214 to the first hydrophilic ferromagnetic particles as shown in FIG.3.

In this way, dispersion degree of the ferromagnetic particle having hydrophilic character can be increased and the highly dispersed state can be stabilized. Polymer coated ferromagnetic particles having further desirable dispersion degree, and further good polymer coating with sharp particle size distribution can be obtained by proceeding the emulsion polymerization of the emulsion liquid 260 prepared using the ferromagnetic particles having hydrophilic character.

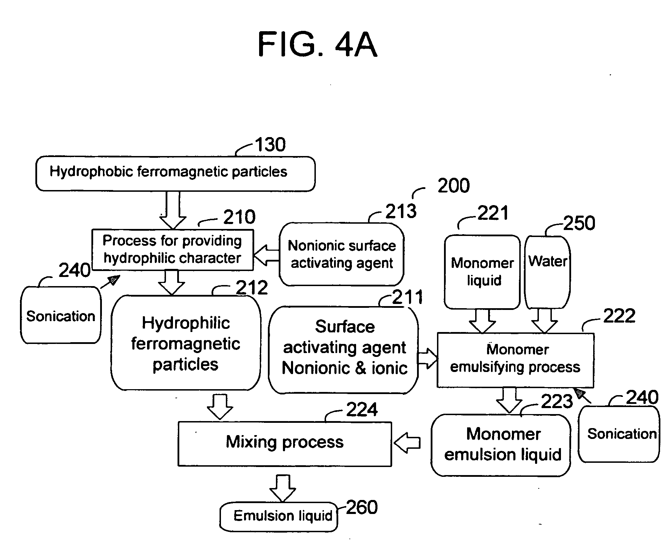

The emulsifying process 200 shown in FIG.1 can have a constitution as shown in FIG.4A. At the emulsifying process 200 shown in FIG.4A, the dispersion liquid of the ferromagnetic particles having hydrophilic character 212 is obtained by providing hydrophilic character to the hydrophobic ferromagnetic particles adding water solution of surface activating agent 213 having nonionic hydrophilic group to hydrophobic ferromagnetic particles 130. At the monomer emulsifying process 222, on the other hand, monomer emulsion liquid 223 is obtained adding water solution of nonionic surface activating agent and ionic surface activating agent 211 to the monomer liquid 221 and mixing and emulsifying adding water 250 as needed. The process can be constructed such-that the emulsion liquid 260 is obtained by mixing the dispersion liquid of the ferromagnetic particles having hydrophilic character 212 and the monomer emulsion liquid 223 at the mixing process 224. In each of these emulsifying process, sonication 240 giving ultrasonic vibration to liquid can be applied.

As a result of emulsion polymerizing the polymerized liquid 260 in this way, polymer coated ferrite particles having desirable polymer coating to each of the hydrophobic ferromagnetic particles 130 and having desirable dispersion characteristics can be obtained.

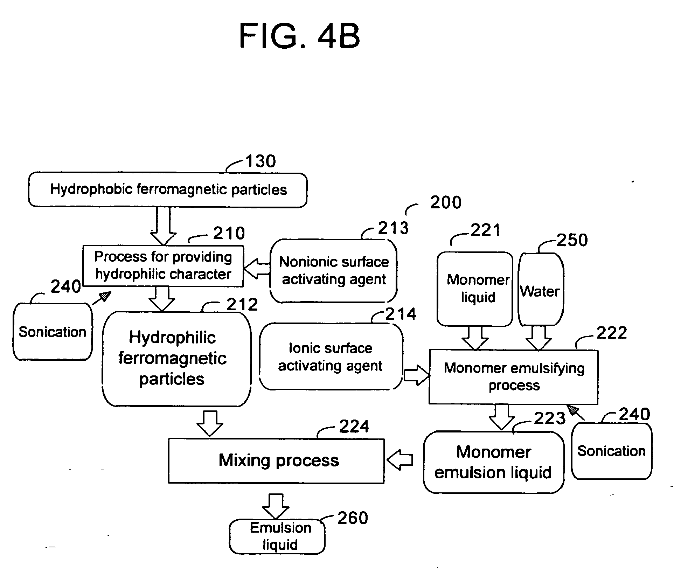

As shown in FIG.4B, nonionic surface activating agent 213 is used similar to the case for FIG.4A at the process for providing hydrophilic character 210 in the emulsifying process 200 of providing hydrophilic character to the hydrophobic ferromagnetic particles 130. For emulsifying monomer liquid 221, on the other hand, the ionic surface activating agent 214 can be used. In this way, as a result of emulsion polymerizing the polymerized liquid 260, polymer coated ferrite particles having desirable polymer coating to each of the hydrophobic ferromagnetic particles 130 and having desirable dispersion characteristics can be obtained.

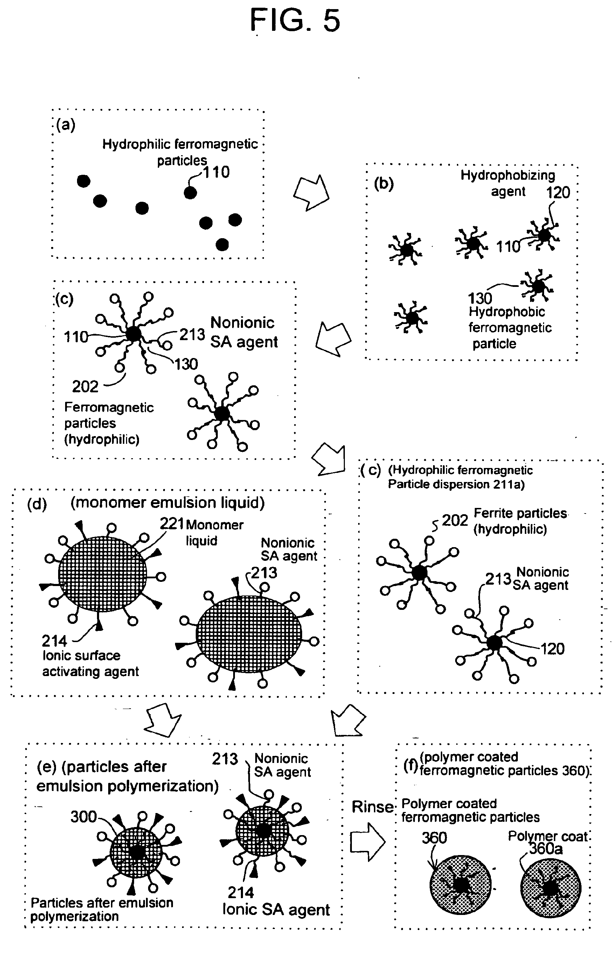

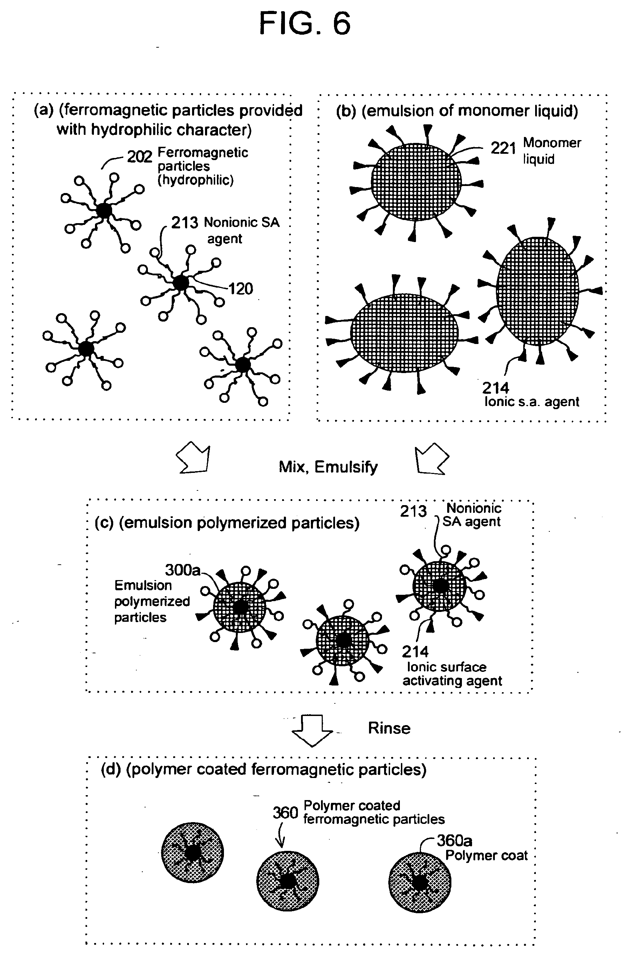

FIG.5 and FIG.6 are figures schematically showing an embodiment for polymer coating to ferromagnetic particles.

Processing the hydrophilic ferromagnetic particles 110 shown (a) in FIG.5 with an aliphatic acid as the hydrophobizing material 120 as shown (b) in FIG.5 to hydrophilic ferromagnetic particles 110 shown (a) in FIG.5, hydrophobic ferromagnetic particles 130 hydrophobized by using hydrophobizing material 120 are obtained.

Dispersion liquid in which hydrophilized ferromagnetic particles 202 are dispersed in water as shown schematically (a) in FIG. 5 is obtained by immersing and dispersing the hydrophobic ferromagnetic particles 130 in water solution of nonionic surface activating agent 213.

As shown (c) in FIG. 5, dispersion liquid of hydrophilic ferromagnetic particles dispersing ferromagnetic particles having hydrophilic ferromagnetic character 202 and emulsion liquid 221 shown (d) in FIG. 5 are mixed, i.e., the emulsion liquid 221 prepared by mixing monomer liquid having water as continuous phase using nonionic surface activating agent 213 and ionic surface activating agent 214. The mixture is emulsified giving ultrasonic vibration as sonication. The obtained emulsion is then heated up to 50 to 90°C, desirably to 60 to 80°C, and emulsion was polymerized adding and mixing initiator. Then emulsion polymerized particles 300a shown schematically (e) in FIG. 5 are obtained. After removing nonionic surface activating agent 213 and ionic surface activating agent 214 from the emulsion polymerized particles 300a by rinsing, polymer coated ferromagnetic particles 360 coated with polymer coat 360a as shown schematically (f) in FIG.5 are obtained. According to this procedure, polymer coated ferromagnetic particles having desirable dispersibility, coating quality and sharp particle diameter distribution can be obtained.

The emulsion polymerization can be performed using nonionic surface activating agent solution 213 to obtain hydrophilic property of the hydrophobic ferromagnetic particles 130 at one hand as shown (a) in FIG.6, and using only surface activating agent 214 having hydrophilic group do obtain monomer emulsion liquid 221, at the other hand, as shown (b)in FIG6. According to this procedure, polymer coated ferromagnetic particles 360 having desirable dispersibility, polymer coat 360a with desirable coating quality and sharp particle diameter distribution can be obtained.

### (2) Ferromagnetic Particles

As the ferromagnetic particles applied to these ferromagnetic particles, magnetite (Fe₃O₄), maghemite (γ-Fe₂O₃), and particles having intermediate composition of these particles can be used. In addition, ferrite particles in which the characteristics of the particle are controlled adequately for each purpose replacing a part of Fe elements in magnetite particles by other element such as Li, Mg, Mn, Co, Ni, Cu and Zn, can be used. For example, larger magnetization can be obtained by replacing part of Fe in ferrite such as magnetite by Mn or Zn. Further, metal particles having ferromagnetic characteristics such as Fe, Co, and Ni can be applied instead of ferrite particles. Particles having larger saturation magnetization per volume can be obtained by using metal alloys or inter-metallic compounds. Inorganic ferromagnetic particles can be applied utilizing their advantageous characteristics. It is essentially important to achieve resistance to corrosion for applying these particles. When Fe particles are used, for example, the surfaces are desirable to be covered with stable oxide such as magnetite. Polymer coating of these Fe particles can be obtained using the method similar to the case for polymer coating to magnetite particles.

For the purpose of handling polymer coated ferromagnetic particles easily using magnetic field, the particles are desirable to show ferromagnetic character at temperature at which the coated particles are used. When the ferromagnetic particles are ferrite particles, for example, the particle average diameter of the ferrite particles is desirable to be 30 nm or more. From the point of ensuring sufficient dispersion stability of the ferrite particle dispersion liquid, however, the particle average diameter of the ferriteparticles is desirable tobe 300 nmor less and more desirable to be 100 nm or less. The average diameter of ferrite particles in the present invention described above is obtained calculating-arithmetic average of the measured diameter values of 500 particles obtained by use of transmission electron microscope photographs.

### (3) Material for providing hydrophobic character

As the agent material for providing hydrophobic character to ferromagnetic particles having hydrophilic character, materials adsorbing tightly at the surface of the ferromagnetic particles and providing hydrophobic character to ferromagnetic particles with abrasion resistive at treatments such as water treatment are desirable. According to the present invention, an aliphatic acid showing tight adsorption character is used for this purpose. The aliphatic acid can be adsorbed to ferromagnetic particles by contacting the aliphatic acid water solution to the ferromagnetic particles and adjusting pH value of the solution.

The aliphatic acid is desirable to carry functional group having capability of radical polymerization reaction. When the aliphatic acid carries functional group having capability of radical polymerization, co-polymerization between the aliphatic acid and the monomer takes place. Then sufficient and stable polymer coats can obtained that coat the ferromagnetic particles. As the atomic groups having capability of radical polymerization, atomic groups having double bond between carbon atoms at the terminal such as vinyl group (CH₂=CH-), methacryl group (CH₂=C(CH₃)-CO-) can be cited.

When aliphatic acid carrying double bond between carbon atoms at the terminal of hydrophobic group and carrying no other double bond between other carbon atoms is applied, the number of carbons is desirable to be from 6 to 18. The number of carbons more desirable to be from 8 to 15, and further more desirable to be from 10 to 15. Of these aliphatic acids, 10-undecenoic acid is one of the materials for providing hydrophobic character applied in the present invention. When aliphatic acid carrying double bond between carbon atoms at the terminal of hydrophobic group side and further carrying other double bond between carbon atoms is used as the material for providing hydrophobic character to obtain hydrophobic ferromagnetic particles, the number of carbons is desirable to be from 6 to 20, more desirable to be from 10 to 20, further desirable to be from 12 to 18.

In recent years, methods of synthesizing ferrite particles in organic solvents, represented by polyol reduction method and inverse micelle method, have been generally used as described in Sun S. et al. J. Am. Chem. Soc. (2004), 126(1); 273-279, and Jongnam P et al. Nature Mater. (2004) 3 891-895. Ferrite particles using these methods show hydrophobic character having reactant material adsorbed already at the surface of the ferrite particles after the synthesis reaction. Of course the ferrite particles synthesized using these methods can be available as the hydrophobic ferrite particles of the present invention.

### (4) Surface activating agent

In the present invention, nonionic surface activating agent and ionic surface activating agent can be used as the surface activating agent for providing hydrophilic character by adsorbing the agent to hydrophobic ferromagnetic particles that are provided with hydrophobic character for providing the agent, for emulsifying adding emulsifying monomer liquid, and for polymerizing the emulsion. As the surface activating agent for the purpose, adequate combination of nonionic surface activating agent and ionic surface activating agent is used.

As the nonionic surface activating agent having nonionic hydrophilic group used in the present invention, various surface activating agent having polyethylene oxide (PEO) chains, or the one having PEO chains with partially replaced by oxypropylene or oxybutylene chains can be desirably applied. As the surface activating agents having nonionic hydrophilic group, polyoxyethylene alkylether (product name: Emulgen (supplied by KaoCo.)) and nonylphenol polyethoxylate Trinton X-405 can be used.

These nonionic surface-activating agents can be used as water solution at a concentration of 70%, for example. The quantity of addition is therefore desirable to be at the rate of 0.012 g or more per 1 g of monomer. The quantity is more desirable to be at the rate of 0.047 g or more per 1 g of monomer. Too much addition of surface activating agent, however, is not desirable because polymer-coating formation becomes difficult. Therefore, the quantity of addition thereof is desirable to be 0.23 g or less and is more desirable to be 0.1 g or less per 1 g of monomer.

Of ionic surface activating agents, various surface activating agents having anionic hydrophilic group such as sulfonic acid group, sulfuric acid group, phosphoric acid group, or polyphosphoric acid group can be used in addition to the surface activating agent having carboxyl group such as aliphatic acid. As surface activating agent having carboxyl group, aliphatic acids carrying 11-15 carbons with straight chain alkyl group can be desirably used. As surface activating agents having cationic hydrophilic atomic group, various surface activating agents having amino group such as long chain primary amine, secondary amine, various tertiary amine, and quaternary ammonium ion. Similarly, surface activating agents having unsaturated bond in the alkyl chain can also be used desirably. The hydrophobic group of the ionic surface activating agent is desirable to be a long chain having prescribed length or more to maintain micelles of the emulsion stable in the process of emulsion polymerization process. For example, the molecule of linear chain type primary amine CₙH₂ₙ₊₁NH₂ is desirable to have long chain of n being 10 or more, and the molecule is further desirable to have chain of n being 11 or more. On the other hand, when n is too large, the function as the agent for forming emulsion is not sufficient. Therefore, the n value is desirable to be 17 or less, and the n value is more desirable to be 15 or less, such as the molecule, for example, aminoundecane.

It is difficult to determine the quantity of adding the ionic surface-activating agent, because the quantity depends on the purpose and the size of the polymer coated ferromagnetic particles. Here, only outline of the desirable quantity is described. An example of the desirable quantity is 1.5 µmol or more, and the more desirable quantity is 6 µmol or more. On the other hand, the quantity of the ionic surface-activating agent is desirable to be less than 50 µmol or equal to 50 µmol, and is more desirable to be less than 16.5 µmol or equal to 16.5 µmol.

### (5) Monomer and organic solvent for forming monomer solution

The most suitable monomer used for the present invention can be chosen from various monomers carrying functional group having capability of radical polymerization reaction on its purpose. As these monomers carrying functional group having capability of radical polymerization reaction: aromatic vinyl compounds including styrene, α-methyl styrene, o-vinyl toluene, m-vinyl toluene, p-vinyl toluene and divinyl benzene; unsaturated carboxylic acids including (meta) acrylic acid and crotonic acid; (meta) acrylates including methyl(meta) acrylate, ethyl(meta) acrylate, n-propyl (meta) acrylate, i-propyl (meta) acrylate, n-butyl (meta) acrylate, t-butyl (meta) acrylate, n-hexyl (meta) acrylate, 2-ethylhexyl(meta) acrylate, (poly)ethylene glycol di(meta)acrylate, (poly) polypropyleneglycol di(meta)acrylate, trimethylol propane tri(meta)acrylate, glycidyl(meta)acrylate; vinyl cyanide compounds including (meta) acrylonitrile, vinylidene cyanide;Vinyl halide compounds including vinyl chloride, vinylidene chloride, vinyl floride, vinylidene floride, tetra fluoroethylene can be cited. Of these monomers, aromatic vinyl compounds and (meta) acrylates of these monomers can especially be used desirably. These monomers can be used as one type or as mixture of plural types. At least one type of these monomers used is desirable to have functional group that can bond to other material placed at the polymer coated surf ace after polymer coating. When a combination of styrene and glycidylmetacrylate (GMD) is used at the surface of the formed coating, glycidyl group (epoxy group) can be placed at the surface of the coating, for example and various compounds including biologically active substance through the functional group.

various organic solvents can be used as organic solvents for solving these monomers to obtain monomer solution. For example, alkanes having 10-20 carbon atoms per molecule, diethyl ether and so on can be used. Diethyl ether shows hydrophobic character due to the two ethyl groups of the molecule, although the ether has hydrophilic ether bond. Adding surface activating agent in water, emulsion particles of diethyl ether can be obtained. So, the diethyl ether can desirably be used as organic solvent for monomer solution liquid. The monomer for use can be solved into organic solvent in a predetermined composition ratio and can be used as monomer mix adding hydrophobic initiator to the solution as needed. Composition of the monomer mix can be controlled such that desirable polymer coated ferromagnetic particles are formed by emulsion polymerization.

### (6) Initiator and cross linking agent

In order to carry out radical polymerization of the monomer, the emulsion of the monomer mix is heated and hydrophilic initiator is added and mixed to the continuous water phase of the emulsion. Hydrophobic initiator also can be used by adding and mixing to the monomer.

As water solve hydrophilic initiators, peroxides including benzoyl peroxide, potassium peroxodisulfate (KPS), and ammonium peroxodisulfate (APS); and water soluble azo compounds including V-50 (product name, supplied by WAKE Co., (2,2'-Azobis(2-methylpropionamidine) dihydrochloride, (2, 2'-Azobis(2-amidinopropane) dihydrochloride)) can be used. As hydrophobic initiators, various azo-compounds such as azobisisobutyronitrile (AIBN) can be used.

As other initiators, azo-compounds including VA-080 (product name, by WAKO Co., (2,2'-Azobis{2-methyl-N-[1,1-bis(hydroxymethyl)-2-hydroxyeth yl] propionamide), VA-085 (product name, by WAKO Co., (2,2'-Azobis{2-methyl-N-[2-(1-hydroxybuthyl)]-propionamide}), VA-086 (product name, by WAKO Co., (2,2'-Azobis[2-methyl-N-(2-hydroxyethyl)-propionamide]), VA-057 (product name, by WAKO Co., (2,2'-Azobis (N-(2-carboxyethyl) -2-methyl-propionamidine, (2,2'-Azobis{2-[N-(2-carboxyethyl) amidino]propane}), V-501 (product name, by WAKO Co., (4,4'-Azobis(4-cyanovaleric acid), (4,4'-Azobis(4-cyanopentanoic acid)), VPE-0201(product name, by WAKO Co. ), and VPE-0601 (product name, by WAKO Co.) can be used.

As cross-linking agent, at least either one of cross-linking agents including divinyl benzene, triarylamine, 1,3,5-triacryloil-hexahydro-s-triazine, trimecin acid triaryl, and ethyleneglycol dimethaclilate can be used.

### (7) Polymer coated ferromagnetic particles

Several examples of the polymer coated ferromagnetic particles obtained in these procedures are explained.

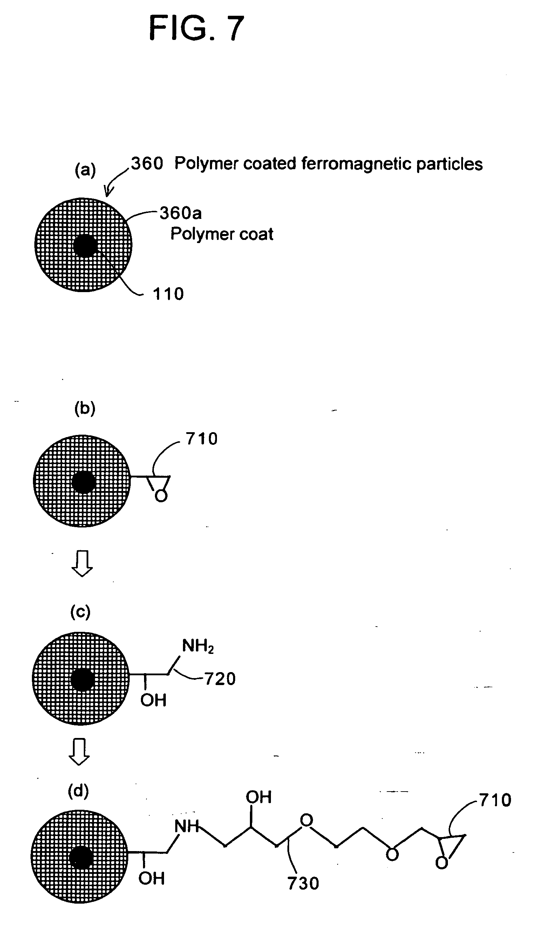

FIG. 7 illustrates schematically an embodiment of the polymer coated ferromagnetic particles according to the present invention. The (a) of FIG. 7 is a figure showing the polymer coated ferromagnetic particles 360a, monomer forming coating and polymerizing ferromagnetic particles 110 through material providing hydrophobic character adsorbed to the Hydrophilic ferromagnetic particles.

The (b) of FIG.7 shows 10-undecenoic acid adsorbed to the hydrophilic ferromagnetic particles 110 and co-polymerized with styrene and glycidyl metacrylate coating the hydrophilic ferromagnetic particles 110. At the surface of the coated particles, many terminals of epoxy group 710 are placed. Actually, many terminals of epoxy group 710 are existed on the surface of each particle, schematically only one terminal is shown for simplicity. The (c) of FIG.7 shows one of the particles opening the glycidyl group (epoxy group) and providing hydroxy group terminals and amino group 720 terminals by treating the particles shown (b) in FIG.7 with ammonium. The (d) of FIG.7 shows the particles providing spacer 730 by bonding monoethylene glycol glycidylether or polyethylene glycol glycidylether (EGDE) molecule to the amino group 720 shown (c) in FIG. 7. The particles can be applied as carrier of affinity chromatography. Providing epoxy group 710 at the top end of the spacers 730 that bond biological molecules, The particles can easily bond various molecules avoiding steric hinderance.

When the particles of the polymer coated ferromagnetic particles are ferrite particles, the particle can be used for various applications by choosing the diameter size of within 25-400 nm because the magnetization can be obtained at suitable values for handling using magnetic field. For use as media for performing magnetic separation of biological molecules, for example, it is desirable that the average ferrite particle diameter of 30-100 nm and the average polymer coated particle diameter of 35-150 nm with weight ratio of the magnetic particles to 1/10 or more, and more desirable to be 1/4 or more to ensure magnetic attractive force of the particles. These polymer coated ferromagnetic particles are desirable to have uniformity in their particle sizes. When the particle size uniformity are lost, measurement results using the particles loose their reproducibility. For this reason, standard deviation of the particle distribution is desirable to be no more than 50 percent of the average particle diameter and more desirable to be no more than 10 per cent. The average diameter is arithmetic average diameter averaged to number of the particles.

The organic solvent durability of the polymer coating can be increased by cross-linking the polymer using cross-linking agent. The organic solvent durability can be increased further by applying the EGDE modification to the polymer coating.

These particles according to the present invention can be used as media for magnetic handling in biological science application, medical applications and various industrial application, because each particle of these polymer coated ferromagnetic particles can be separated each other having uniform particle size and shows desirable response tomagnetic field. Many applications of the particles making best use of the particle characteristics of the particles are expected such as magnetic colloid application showing excellent magnetic field response and stability compared with magnetic colloids using known polymer coated ferromagnetic particles.

It is found that the polymer coated ferromagnetic particles according to the present invention can be fine particles with excellent organic solvent durability. The polymer coating can be not solved by organic solvents and can keep their shapes even if the particles are immersed in organic solvents. The solvent durability can increase by cross-linking the polymer. Increase of the organic solvent durability can also be obtained by providing spacer such as EGDE.

The polymer coated ferromagnetic particles having such constitution can be used for various applications such as carrier particles of affinity chromatography for chemical materials including proteins, peptides, nucleic acid and drugs, magnetic solid phase magnetic carrier particles for combinatorial chemistry, as the magnetic solid carrier particles for affinity chromatography, and as magnetic solid phase carrier particles for chemical synthesis of peptide or nucleic acid. For example when the particles are used as magnetic carrier of affinity chromatography, the availability of the particles in organic solvent allows bonding water insoluble compounds to the coating of the ferromagnetic particles quantitatively as ligands in organic solvent.

As a method for detecting biological molecules including nucleic acids, methods using a magnetic sensor are known. As the magnetic sensors, giant magnetoresistance effect device (GMR), tunnel magnetoresistance effect device (TMR), hall sensor, and SQUID type flux meter are cited. Bio-molecules are fixed on the device and are also fixed on he magnetic particles. A magnetic signal can be obtained only when there are interactions between these bio-molecules. The polymer coated ferromagnetic particles according to the present invention can be spherical particles with narrow particle size distribution. Since the particles can be homogeneous and each particle can carry similar number of ferromagnetic particles, the particles are useful as an excellent-magnetic marker to bio-molecules of a magnetic sensor system.

### [Example 1]

100 µl of 10-undecenoic acid is added to 150 mg of ferromagnetic ferrite particles having average particle diameter of 40 nm obtained by precipitating from water solution and the 10-undecenoic acid was adsorbed in saturation to the ferromagnetic particles. Remained 10-undecenoic acid that did not adsorbed to the particles was eliminated by rinsing. Then hydrophobic ferromagnetic particles were obtained.

Nonionic surface activating agent water solution containing 0.3 g of Emulgen 1150S-70 (produced by KAO Co.) having chemical formula 1 and carrying PEO chain was added to the hydrophobic ferromagnetic particles and adsorbed the nonionic surface activating agent to the ferromagnetic particles having hydrophilic character, and ultrasonic vibration was applied as sonication. Then colloidal water liquid of ferrite particles having hydrophilic character was obtained.

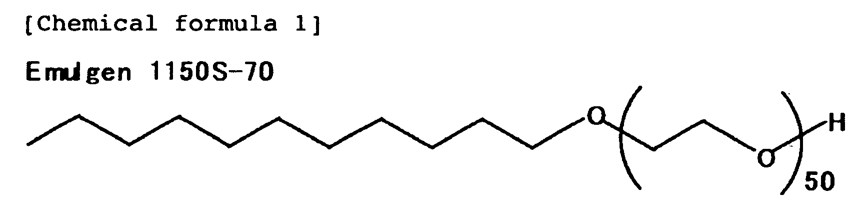

The water solution dissolving 10 µl of ionic surface activating agent aminoundecane solved in 56 µl of 1 M HCl was added to the colloidal water liquid of ferrite particles having hydrophilic character. Then a state of coexisting nonionic surface activating agent and ionic surface activating agent at the surface of ferrite particle colloid having hydrophilic character was established.

Then emulsion liquid was obtained by adding monomer mix containing 2.7 g of styrene (monomer), 0.3 g of GMA (glycidyl metacrylate, monomer), 0.025 g of AIBN (azobisisobutylonitrile, initiator), 0.08 g of DVB (divinylbenzene, cross linking agent) and 2.5 g of diethylether to the colloidal liquid and applying ultrasonic vibration as sonication.

Then water was added to the emulsion liquid such that the total liquid quantity of 125 g and applied ultrasonic vibration of sonication. The liquid was then heated under mixing at rotation speed of 350 rpm. When the temperature of the liquid attained at 70°C about 20-30 minutes after beginning of the heating, water soluble initiator V-50 (product of WAKO PURE CHEMICAL Co.) was added and polymerization reaction was proceeded for 12 hours.

Polymer coated ferrite particles were obtained after rinsing the emulsion polymerized particles. As a result of electron microscope observation using a transmission electron microscope, the obtained polymer coated ferrite particles were monodispersed particles carrying 1-3 ferrite particles inside each particle having average particle diameter of 163 nm with the diameter standard deviation of 20 nm.

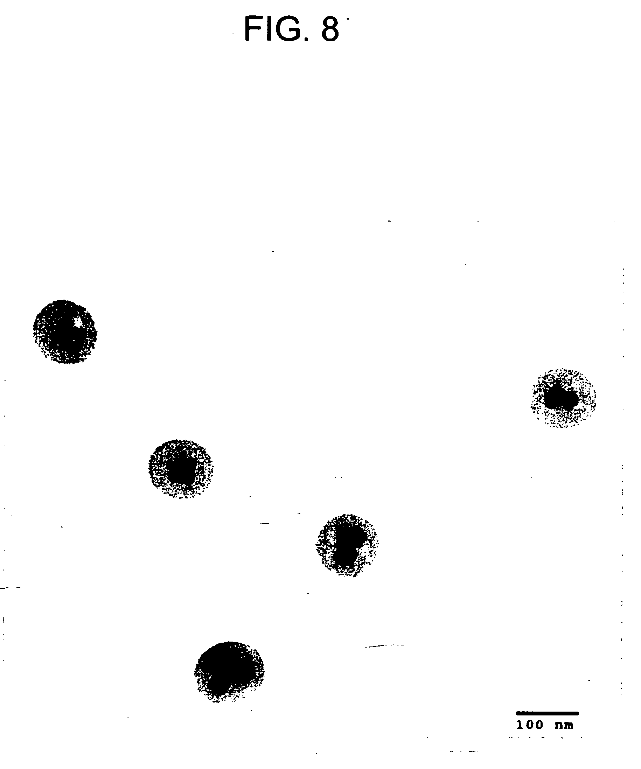

FIG. 8 shows an example of electron microscope photographs for the prepared polymer coated ferrite particles using this preparation method.

The polymer coated ferrite particles showed strong magnetization and the particles could be easily attracted by magnetic field gradient caused by using magnet and so on.

### [Example 2]

coated ferrite particles were obtained by using the process described in the Example 1, except replacing nonionic surface activating agent by 0.3 g of Triton X-405 given by [Chemical equation 2] having PEO chain, replacing the ionic surface activating agent by 10 µl of 10-undecenoic acid dissolved in 56 µl of 1 M NaOH solution, and replacing the water soluble initiator by 25 mg of KPS.

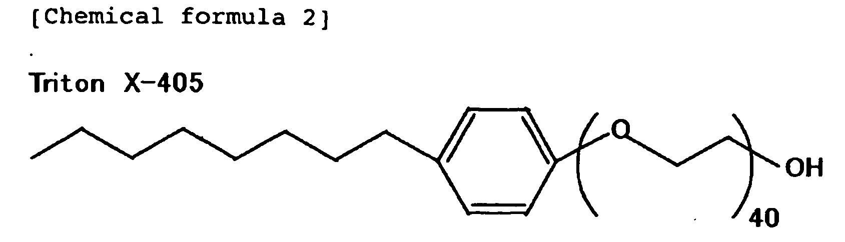

As a result of observing the obtained polymer coated ferrite particles using the transmission electron microscope, it was found that the particles were monodispersed particles carrying 1-3 ferrite particles inside each particle and having average particle diameter of 125 nm.

### [Example 3]

Polymer coated ferrite particles were obtained using the process condition described in the Example 1, except changing the quantity of V-50 addition to 100 mg. As a result of observing the obtained polymer coated ferrite particles using the transmission electron microscope, it was found that the particles were monodispersed particles carrying 1-5 ferrite particles inside each particle having average particle diameter of 153 nm with the diameter standard deviation of 22 nm.

### [Example 4]

Polymer coated ferrite particles were obtained using the process condition described in the Example 3, except changing the quantity of nonionic surface activating agent Emulgen 1150S-70 addition to 0.5 g. As a result of observing the obtained polymer coated ferrite particles using the transmission electron microscope, it was found that the particles were monodispersed particles carrying 1-4 ferrite particles inside each particle having average particle diameter of 90 nm with the diameter standard deviation of 22 nm.

### [Example 5]

Polymer coated ferrite particles were obtained using the same composition and process condition described in the Example 1, except replacing the ferrite particles by ferrite particles having average diameter of 70 nm. As a result of transmission electron microscope observation for the obtained polymer coated ferrite particles, it was found that the particles were monodispersed particles carrying 1-3 ferrite particles inside each particle having average particle diameter of 200 nm with the diameter standard deviation of 30 nm.

### [Example 6]

Polymer coated ferrite particles were obtained by using the same composition and process condition described in the Example 1, except using processes of obtaining emulsion liquid having water as continuous phase of : obtaining ferrite particle water dispersion by dispersing ferrite particles having hydrophobic character using 0.2 g of nonionic surface activating agent Emulgen 1150S-70; obtaining emulsion liquid mixing the monomer mix containing monomer, organic solvent, initiator, and cross linker agent dissolving 10 µl aminoundecane as surface activating agent dissolved in 56 µl of 1 M HCl solution and adding 0.1 g of nonionic surface activating agent Emulgen 1150S-70 applying ultrasonic vibration of sonication; and mixing the ferrite particle water dispersion and the emulsion liquid.

As a result of transmission electron microscope observation for the obtained polymer coated ferrite particles, it was found that the particles were monodispersed particles carrying 1-5 ferrite particles inside each particle having average particle diameter of 175 nm with the diameter standard deviation of 12 nm.

### [Example 7]

Polymer coated ferrite particles were obtained using the same composition and process condition described in the Example 1, except using processes of forming emulsion liquid having water as continuous phase of: obtaining ferrite particle water dispersion prepared by dispersing ferrite particles having hydrophobic character using 0. 3 g of nonionic surface activating agent Emulgen 1150S-70; obtaining monomer liquid obtained by dissolving 10 µl aminoundecane dissolved in 56 µl of 1 M HCl solution to polymer mix containing monomer,organic solvent, initiator and cross linker agent, mixing with water and giving ultrasonic vibration as sonication; and mixing the ferrite particle water dispersion and the monomer liquid.

As a result of transmission electron microscope observation for the obtained polymer coated ferrite particles, it was found that the particles were monodispersed particles carrying 1-3 ferrite particles inside each particle having average particle diameter of 120 nm with the diameter standard deviation of 12 nm.

The conditions and results of the Examples 1-7 are shown in Table 1 and Table 2.

**[Table 1]**

| | | Example 1 | Example 2 | Example 3 |
|---|---|---|---|---|
| Ferrite particles | Average diameter (nm) | 40 | 40 | 40 |
| | Standard deviation (nm) | 10 | 10 | 10 |
| Hydrophobizing | agent | 10-undecenoic acid | 10-undecenoic acid | 10-undecenoic acid |
| Surface activating agent (SA) | | | | |
| Nonionic | | Emulgen 1150S-70 (KAO Co.) 0.3 g | Triton X-405 (Sigma Co.) 0.3 g | Emulgen 1150S-70 (KAO Co.) 0.3 g |
| Ionic | | Aminoundecane 10 µl 1M HCl 56 µl | 10-undecenoic acid 10 µl 1M NaOH 56 µl | Aminoundecane 10 µl 1M HCl 56 µl |
| Monomer mix | | Styrene 2.7 g GMA 0.3 g, Diethylether 2.5 g AIBN 0.025 g Cross linking agent Divinylbenzene 0.08 g | Styrene 2.7 g GMA 0.3 g, Diethylether 2.5 g Cross linking agent Divinylbenzene 0.08 g | Styrene 2.7 g GMA 0.3 g, Diethylether 2.5 g Cross linking agent Divinylbenzene 0.08 g |
| Emulsifying process | | 1st hydrophilic -> 2nd hydrophilic -> -> Mix. monomer | 1st hydrophilic -> 2nd hydrophilic -> -> Mix. monomer | 1st hydrophlic -> 2nd hydrophlic -> -> Mix. Monomer |
| Hydrophilic initiator | | V50 50mg/5ml | KPS 25mg | V50 100mg |
| Polymer coated ferrite particles | Average diameter (nm) | 163 | 125 | 153 |
| | Standard deviation (nm) | 20 | 15 | 22 |
| | Number of particles | 1-3 | 1-3 | 1-5 |
| | Magnetic property | Magnetic field response: good | Magnetic field response: good | Magnetic field response: good |

**[Table 2]**

| | | Example 4 | Example 5 | Example 6 | Example 7 |
|---|---|---|---|---|---|
| Ferrite Particles | Average diameter(nm) | 40 | 70 | 40 | 40 |
| | Standard deviation(nm) | 10 | 20 | 15 | 15 |
| Hydrophobizing agent | | 10-undecenoic acid | 10-undecenoic acid | 10-undecenoic acid | 10-undecenoic acid |
| Surf. Activ. Agent. (SA) | | | | | |
| Nonionic | | Emulgen 1150S-70 (KAO Co.) 0.5 g | Emulgen 1150S-70 (KAO Co.) 0.3 g | Emulgen 1150S-70 (KAO Co.) 0.3 g | Emulgen 1150S-70 (KAO Co.) 0.3 g |
| Ionic | | Aminoundecane 10 µl 1M HCl 56 µl | 10-undecenoic acid 10 µl 1M HCl 56 µl | Aminoundecane 10 µl 1M HCl 56 µl | Aminoundecane 10 µl 1M HCl 56 µl |
| Monomer mix | | Styrene 2.7 g GMA 0.3 g, Diethylether 2.5g AIBN 0.025 g Cross linking agent Divinylbenzene0.08g | Styrene 2.7 g GMA 0.3 g, Diethylether 2.5 g Cross linking agent Divinylbenzene0.08g | Styrene 2.7 g GMA 0.3 g, Diethylether 2.5g AIBN 0.025 g Crosss linking agent Divinylbenzene0.08g | Styrene 2.7 g GMA 0.3 g, Diethylether 2.5g AIBN 0.025 g Cross linking agent Divinylbenzene0.08g |
| Emulsifying process | | 1st hydrophilic -> 2nd hydrophilic -> -> Mix. Monomer | 1st hydrophilic -> 2nd hydrophilic -> -> Mix. monomer | Mixing: Ferrite dispersion. with nonionic SA and monomer with nonionic and ionic SA | Mixing Ferrite dispersion. with nonionic SA and monomer with ionic SA |
| Hydrophilic initiator | | V50 100mg | V50 50mg/5ml | V50 50mg/5ml | V50 50mg/5ml |
| Polymer coated Ferrite particles | Average diameter(nm) | 90 | 200 | 175 | 120 |
| | Standard deviation (nm) | 22 | 30 | 12 | 10 |
| | Number of particles | 1-4 | 1-3 | 1-5 | 1-3 |
| | Mag. property | Mag. field response: good | Mag. field response: good | Mag. field response: good | Mag. Field response: good |

### [Example 8]

Organic solvent durability test was performed for the polymer coated ferrite particles of the Example 1. Particles are examined after the test condition of immersing in solvent for 12 hours. As the result, the polymer coatings after immersion were maintained for organic solvents of methanol, ethanol, isopropanol, tetrahydrofuran, acetonitrile, acetic ether, dioxane, N, N-dimethylformamide, dimethylsulfoxide, acetone, diethylether, toluene, dichloromethane, chloroform and hexane, and durability to these organic solvents was proved.

### [Example 9]

Modification of bonding EGDEs as spacers for the polymer coated ferrite particles manufactured in the Example 1 was performed.

First of all, epoxy groups of GMAs were reacted adding NH₄OH solution and adjusting pH using HCl solution. Then the epoxy groups of GMAs were opened.

Excess EGDE molecules were supplied to the amino groups appeared as a result of opening to the polymer coated ferrite particles and stirred adjusting pH with NaOH water solution, and combined a epoxy group of a EGDE molecule to a amino group of the polymer coated ferrite particles. Supplying EGDE molecules in excess, bonding formation of epoxy groups at both sides of an EGDE molecule with amino groups of polymer coated ferrite particles was avoided. After the bonding reaction, the particles were rinsed with water applying handling technique of magnetic separation. Then modified polymer coated ferrite particles having EGDE spacers were obtained.

Organic solvent durability test was performed to the polymer coated ferrite particles having EGDE modification. The test condition is the same as explained in the Example 7 of immersing for 12 hours in each solvent. As the result, the polymer coatings after immersion were maintained for organic solvents of methanol, dioxane, DMF (dimethylformamide) and DMSO (dimethylsulfoxide), and durability of the particles to these organic solvents was proved.

Furthermore, organic solvent durability tests of the EGDE modified polymer coated ferrite particles were conducted for solvents of diethylether, acetone, toluene, chloroform and hexane respectively under the same condition as explained above. As the result, the polymer coatings after immersion were maintained for each of these organic solvents, and durability to these organic solvents was also proved.

### (Comparative example 1)

Preparation of polymer coated ferrite particles were tried using the process described in the Example 6, except using only 0.3 g of water solution of nonionic surface activating agent Emulgen 1150S-70 (produced by KAO Co.), and not using ionic surface activating agent. However, the obtained polymer coated ferrite particles were polydisperse particles lacking homogeneity. It was shown that this condition of Comparable example 1 is insufficient to form homogeneous monodisperse polymer coated ferrite particles.

### (Comparative example 2)

Preparation of polymer coated ferrite particles were tried using the process described in the Example 6, except adding only 20 ml (1%) of ionic surface activating agent dodecyl sodium sulfate and not using nonionic surface activating agent. However, polymer coated ferrite particles were not formed. Then it was found that this condition of Comparable example 2 of using only ionic surface activating agent, and not using nonionic surface activating agent was insufficient for forming polymer coated ferrite particles.

### (Comparative example 3)

Preparation of polymer coated ferrite particles were tried using ferrite particles without providing hydrophobic character instead of the ferrite particles provided with hydrophilic character, and other composition and condition were the same as described in the Example 6. It was shown that this case of Comparative example 3 using ferrite particles without providing hydrophobic character, the condition is insufficient for realizing emulsion polymerization.

### Industrial availability

According to the present invention, polymer coated ferromagnetic particles with very small diameter, having desirable polymer coating with sufficient magnetization for handling the particles using magnetic field can be obtained. Due to these features, the polymer coated ferromagnetic particles can be used for separating biological materials magnetically, for carriers bonding and carrying drugs to the polymer coated ferrite particles and for magnetic markers. Furthermore, wide variety of applications in industrial fields can be expected making use of these distinguishing properties.

## Claims

1. A method of manufacturing polymer coated ferromagnetic particles comprising:
a hydrophobizing process of absorbing hydrophilic groups of an aliphatic acid, said aliphatic acid having hydrophilic groups and hydrophobic groups, onto hydrophilic ferromagnetic particles with an average diameter of 20 to 300 nm so as to obtain hydrophobic ferromagnetic particles;
an emulsifying process of mixing said hydrophobic ferromagnetic particles with a monomer liquid containing a nonionic surface active agent, an ionic surface active agent and a monomer so as to obtain an emulsified liquid through emulsification; and
an emulsion-polymerization process of adding an initiator to said emulsified liquid so as to conduct an emulsion polymerization for said emulsified liquid through radical addition polymerization.

2. The method as claimed in claim 1, wherein the aliphatic acid carries hydrophobic group having a capability of performing an addition polymerization.

3. The method as claimed in claim 1 or 2, wherein the aliphatic acid is 10-undecenoic acid.

4. The method as claimed in any one of claims 1 to 3, wherein the monomer is at least one selected from the group consisting of glycidylmethacrylate and styrene.

5. The method as claimed in any one of claims 1 to 4, wherein the ferromagnetic particles are ferrite particles having an average diameter of 30 to 100 nm.

6. The method as claimed in any one of claims 1 to 5, wherein the nonionic surface-active agent comprises hydrophilic group of polyoxyethylene chain or polyoxyethylene chain replaced in part by at least one selected from the group consisting of oxypropylene and oxybutylene.

7. The method as claimed in any one of claims 1 to 6, further comprising a cross-linking process of adding a cross-linking agent to the monomer liquid in addition to the emulsion polymerization.

8. Polymer coated ferromagnetic particles obtainable by a method according to any one of claims 1 to 7.

9. The polymer coated ferromagnetic particles as claimed in claim 8, having an average particle diameter of 25 to 400 nm, comprising hydrophobic ferromagnetic particles having an average particle diameter of 20 to 300 nm; and a polymer coating the hydrophobic ferromagnetic particles.

10. The polymer coated ferromagnetic particles as claimed in claim 9, wherein the particles comprise residues of the aliphatic acid carrying functional group having a capability of co-polymerizing with a monomer and forming part of the polymer.

## Patentansprüche

1. Verfahren zur Herstellung von polymerbeschichteten ferromagnetischen Teilchen, umfassend:
einen Hydrophobierungsprozess des Absorbierens hydrophiler Gruppen einer aliphatischen Säure, wobei die aliphatische Säure hydrophile Gruppen und hydrophobe Gruppen aufweist, auf hydrophilen ferromagnetischen Teilchen mit einem durchschnittlichen Durchmesser von 20 bis 300 nm, so dass hydrophobe ferromagnetische Teilchen erhalten werden,
einen Emulgierungsprozess des Mischens der hydrophoben ferromagnetischen Teilchen mit einer Monomerflüssigkeit, die ein nicht-ionisches oberflächenaktives Mittel, ein ionisches oberflächenaktives Mittel und ein Monomer enthält, so dass durch Emulgieren eine emulgierte Flüssigkeit erhalten wird, und
einen Emulsionspolymerisationsprozess des Zugebens eines Initiators zu der emulgierten Flüssigkeit, so dass eine Emulsionspolymerisation für die emulgierte Flüssigkeit durch eine radikalische Additionspolymerisation durchgeführt wird.

2. Verfahren nach Anspruch 1, bei dem die aliphatische Säure (eine) hydrophobe Gruppe(n) aufweist, mit der oder denen eine Additionspolymerisation durchgeführt werden kann.

3. Verfahren nach Anspruch 1 oder 2, bei dem die aliphatische Säure 10-Undecensäure ist.

4. Verfahren nach einem der Ansprüche 1 bis 3, bei dem das Monomer mindestens eines ist, das aus der Gruppe, bestehend aus Glycidylmethacrylat und Styrol, ausgewählt ist.

5. Verfahren nach einem der Ansprüche 1 bis 4, bei dem die ferromagnetischen Teilchen Ferritteilchen sind, die einen durchschnittlichen Durchmesser von 30 bis 100 nm aufweisen.

6. Verfahren nach einem der Ansprüche 1 bis 5, bei dem das nicht-ionische oberflächenaktive Mittel hydrophile Gruppen einer Polyoxyethylenkette oder einer Polyoxyethylenkette, die teilweise durch mindestens eines, ausgewählt aus der Gruppe, bestehend aus Oxypropylen und Oxybutylen, ersetzt worden ist, umfasst.

7. Verfahren nach einem der Ansprüche 1 bis 6, das ferner einen Vernetzungsprozess des Zusetzens eines Vernetzungsmittels zu der Monomerflüssigkeit zusätzlich zu der Emulsionspolymerisation umfasst.

8. Polymerbeschichtete ferromagnetische Teilchen, die durch ein Verfahren nach einem der Ansprüche 1 bis 7 erhältlich sind.

9. Polymerbeschichtete ferromagnetische Teilchen nach Anspruch 8, die einen durchschnittlichen Teilchendurchmesser von 25 bis 400 nm aufweisen und hydrophobe ferromagnetische Teilchen mit einem durchschnittlichen Teilchendurchmesser von 20 bis 300 nm und ein Polymer, das die hydrophoben ferromagnetischen Teilchen beschichtet, umfassen.

10. Polymerbeschichtete ferromagnetische Teilchen nach Anspruch 9, wobei die Teilchen Rückstände der aliphatischen Säure umfassen, die (eine) funktionelle Gruppe(n) aufweist, die ein Vermögen zu einer Copolymerisation mit einem Monomer und zum Bilden eines Teils des Polymers aufweist oder aufweisen.

## Revendications

1. Procédé de fabrication de particules ferromagnétiques revêtues d'un polymère, comprenant :
un procédé d'hydrophobisation consistant à absorber des groupements hydrophiles d'un acide aliphatique, ledit acide aliphatique ayant des groupements hydrophiles et des groupements hydrophobes, sur des particules ferromagnétiques hydrophiles présentant un diamètre moyen de 20 à 300 nm, d manière à obtenir des particules ferromagnétiques hydrophobes ;
un procédé d'émulsionnement consistant à mélanger lesdites particules ferromagnétiques hydrophobes à un liquide monomère contenant un agent tensioactif non ionique, un agent tensioactif ionique et un monomère,de manière à obtenir un liquide émulsionné via l'étape d'émulsionnement ; et
un procédé de polymérisation en émulsion consistant à ajouter un initiateur audit liquide émulsionné de manière à effectuer une polymérisation en émulsion pour ledit liquide émulsionné par le biais d'une polymérisation par addition radicalaire.

2. Procédé selon la revendication 1, dans lequel l'acide aliphatique porte un groupement hydrophobe ayant une capacité de participer à une polymérisation par addition.

3. Procédé selon la revendication 1 ou 2, dans lequel l'acide aliphatique est l'acide 10-undécénoïque.

4. Procédé selon l'une quelconque des revendications 1 à 3, dans lequel le monomère est au moins un composé choisi dans le groupe constitué du méthacrylate de glycidyle et du styrène.

5. Procédé selon l'une quelconque des revendications 1 à 4, dans lequel les particules ferromagnétiques sont des particules de ferrite présentant un diamètre moyen de 30 à 100 nm.

6. Procédé selon l'une quelconque des revendications 1 à 5, dans lequel l'agent tensioactif non ionique comprend un groupement hydrophile d'une chaîne de polyoxyéthylène ou d'une chaîne de polyoxyéthylène remplacé en partie par au moins un composé choisi dans le groupe constitué de l'oxypropylène et de l'oxybutylène.

7. Procédé selon l'une quelconque des revendications 1 à 6, comprenant en outre un procédé de réticulation, consistant à ajouter un agent de réticulation au liquide monomère, en plus de la polymérisation en émulsion.

8. Particules ferromagnétiques revêtues d'un polymère que l'on peut obtenir selon l'une quelconque des revendications 1 à 7.

9. Particules ferromagnétiques revêtues d'un polymère selon la revendication 8, présentant un diamètre particulaire moyen de 25 à 400 nm, comprenant des particules ferromagnétiques hydrophobes présentant un diamètre particulaire moyen de 20 à 300 nm ; et un polymère revêtant les particules ferromagnétiques hydrophobes.

10. Particules ferromagnétiques revêtues d'un polymère selon la revendication 9, dans lesquelles les particules comprennent des résidus de l'acide aliphatique portant un groupement fonctionnel ayant une capacité de participer à une co-polymérisation avec un monomère et de faire partie du polymère.
